# EUROPEAN PATENT SPECIFICATION

(11) **EP 2 257 266 B1**
(45) Date of publication and mention of the grant of the patent: **16.03.2016**
(21) Application number: 09734893.2
(22) Date of filing: 23.03.2009
(51) Int. Cl.: A61K 8/27, A61K 8/35, A61K 8/49, A61K 8/67, A61K 8/73, A61Q 17/04

(54) **COMPOSITIONS DESIGNED TO PREVENT/PROTECT AGAINST PHOTOALLERGIC AND PHOTOTOXIC SKIN REACTIONS**
ZUR VORBEUGUNG/ZUM SCHUTZ VOR LICHTALLERGISCHEN UND LICHTTOXISCHEN HAUTREAKTIONEN BESTIMMTE ZUSAMMENSETZUNGEN
COMPOSITIONS MISES AU POINT POUR PRÉVENIR/PROTÉGER CONTRE DES RÉACTIONS CUTANÉES PHOTO-ALLERGIQUES ET PHOTOTOXIQUES

(30) Priority: 01.04.2008 IT MI20080560
(43) Date of publication of application: 08.12.2010
(73) Proprietor: Braguti, Lodovico Gianluca, 23900 Lecco (IT); Varani, Loretta, 40020 Casalfiumanese (BO) (IT)
(72) Inventor: Braguti, Lodovico Gianluca, 23900 Lecco (IT); Varani, Loretta, 40020 Casalfiumanese (BO) (IT)
(74) Representative: Minoja, Fabrizio
(86) International application number: PCT/EP2009/002122
(87) International publication number: WO 2009/129904

(56) References cited:
- WO-A-98/13016
- WO-A-99/33439
- WO-A-2005/004827
- DE-A1- 10 002 725
- DE-A1- 19 933 466
- FR-A- 2 865 398
- US-A- 6 090 369
- US-B1- 7 008 627
- CHATELAIN E ET AL: "PHOTOSTABILIZATION OF BUTYL METHOXYDIBENZOYLMETHANE (AVOBENZONE) AND ETHYLHEXYL METHOXYCINNAMATE BY BIS-ETHYLHEXYLOXYPHENOL METHOXYPHENYL TRIAZINE (TINOSORB S), A NEW UV BROADBAND FILTER" PHOTOCHEMISTRY AND PHOTOBIOLOGY, WILEY-BLACKWELL PUBLISHING, INC, US, vol. 74, no. 3, 1 September 2001 (2001-09-01), pages 401-406, XP001057313 ISSN: 0031-8655

## Description

The present invention relates to compositions for the topical use comprising a combination of systems with a high photoabsorption capacity for ultraviolet A radiation in combination with vitamin A and C derivatives and compounds with a cytoprotective action such as beta-glucan and derivatives thereof, ectoine, and possibly bioflavonoids, proanthocyanidins, polyphenols, verbascosides, xyloglucans or extracts of plant origin containing them.

### Background of the invention

Photoallergy is an inflammatory reaction which occurs in the form of erythema in sensitive individuals after exposure to normally harmless levels of ultraviolet radiation, mainly of the UV-A type.

The action mechanism of photoallergy has long been known¹: the absorption of ultraviolet light produces an excited state of the drug or metabolite, which may follow either of the two routes that eventually leads to photosensitisation.

The first route involves the generation of free radicals. Following the formation of free radicals, the photoproduct can participate in direct electronic transfer or in the covalent bond with key cell components.

The second route involves the generation of a singlet oxygen species which generates oxidation of biomolecules, damaging critical cell components and initiating the release of erythrogenic mediators.

Photoallergy is also mediated by the immune system.

Photoallergy primarily affects individuals with subjective photodynamic intolerances, the most frequent being polymorphic light eruption, an acute, recurrent idiopathic form mainly caused by UV-A rays (and consequently also by exposure through glass), and characterised by polymorphic lesions.

Another cause of photoallergy is pharmaceutical products, especially those which absorb in the UV-A region. Photoallergy can also affect individuals who are not undergoing pharmacological treatments, but come into contact with topical or systemic products which induce phototoxic or photoallergic reactions.

### Examples of photosensitising drugs include:

Anti-infectives such as doxycycline, minocycline, tetracycline, nalidixic acid, flumequine, oxolinic acid, pipemidic acid, pefloxacin, ciprofloxacin, ofloxacin, sulphamethoxazole and griseofulvin; anti-inflammatories such as piroxicam, tiaprofenic acid, naproxen, diclofenac, ibuprofen and ketoprofen; anti-tumorals such as bleomycin, dacarbazine, fluorouracil, methotrexate and vinblastine; psychotropic drugs such as imipramine, amitriptyline, desipramine, clomipramine, thioridazine, chlorpromazine, carbamazepine, chlordiazepoxide and alprazolam; cardiological drugs such as amiodarone, quinidine, captopril, enalapril, hydrochlorothiazide, furosemide, fenofibrate, clofibrate and others, such as benzocaine, promethazine and benzydamine.

Type A ultraviolet radiation is a component of solar radiation characterised by a wavelength of between 320 and 400 nm and the ability to penetrate both the epidermis and the dermis. UV-A rays can therefore cause intense oxidative stress.^{2,3,4,5}

Substances such as diethylamine hydroxybenzoyl hexyl benzoate, zinc oxide and butyl methoxydibenzoylmethane absorb in the UV-A region, and are used both individually and in combination as UV-A filters.

Substances able to guarantee suitable protection of the cutaneous cytoimmunological system and the skin in general against UV-induced oxidative stress have been under study for some time.

Of the various substances studied, glucans have proved particularly interesting. Glucans are polysaccharides characterised by a 1-3 beta-glucoside bond, obtained by biofermentation from yeasts (*Saccharomyces cerevisiae*) and subsequently rendered water-soluble by carboxymethylation (carboxymethyl-beta-glucan). Other similar polysaccharides are extracted from mushrooms.

Glucans^{6,7,8,9,10,11} stimulate the cutaneous immune system and protect it against UV-A induced oxidative stress.

Ectoine^{12,13,14,15,16} is a natural substance with cytoprotective and immunoprotective properties which is present in halophilic bacteria such as *Ectothiorhodospira halochloris.* Said micro-organisms can survive in extreme conditions (salt lakes, sea water and salt deserts exposed to very high doses of UV and IR radiation).

Antioxidant vitamins have also been studied as topical agents to protect against phototoxic damage. For example, vitamin E^{17,18,19,20,21,22} is known to act as a free radical scavenger, also called a "chain-breaking antioxidant". Vitamin C ^{23,24,25, 26,27,28,29} acts (together with other reducing agents like beta-carotene, enzymes such as catalase and superoxide dismutase and compounds such as glutathione) in reduction processes that minimise the damage associated with oxidative phenomena.

Finally, some species of plants such as *Vitis vinifera, Prunella vulgaris*, *Uncaria tormentosa, Paeonia* and *Camellia sinensis* contain substances with a cytoprotective action against damage caused by ultraviolet radiation and oxidative stress. Said plants contain bioflavonoids, proanthocyanidins, polyphenols, verbascosides and xyloglucans as active ingredients ^{30,31,32}.

Topical compositions containing UV-A filters, antioxidant vitamins and either carboxymethyl beta-glucan or ectoine useful for preventing photo-allergic or photo-toxic reactions are known^{35,36,37}.

### Description of the invention

It has been found that the combination of UV-A photoabsorbent systems with antioxidants such as vitamin C and E derivatives and cytoprotective agents such as carboxymethyl beta-glucan and ectoine and possibly bioflavonoids, proanthocyanidins, polyphenols, verbascosides, xyloglucans or plant extracts containing them, is effective, when administered topically, in preventing and protecting against photoallergic and phototoxic skin reactions, by interacting with one another in a particularly advantageous and/or synergic way.

The invention therefore provides topical formulations containing:
a) UV-A photoabsorbent systems;
b) carboxymethyl beta-glucan and ectoine
c) vitamins C and/or E.

The compositions according to the invention perform a preventive and protective activity against photoallergic or phototoxic reactions, mediated by pharmaceuticals, topical products and/or foods, or caused by subjective predispositions.

In order to make systems with a high photoabsorption capacity towards type A ultraviolet radiation, different types of photoabsorbent substances will be used with chemical or physical processes, either individually or in combination.

According to the invention, the UV-A photoabsorbent components will be characterised by a high photofiltration capacity (critical wavelength > 375 nm), and will be chosen in particular from the group comprising diethylamine hydroxybenzoyl hexyl benzoate, zinc oxide, butyl methoxydibenzoylmethane, benzophenones.

According to a preferred aspect of the invention, the photoabsorbent components will be present in the following concentration intervals (of the total components of the composition):
i) diethylamine hydroxybenzoyl hexyl benzoate: 0.5 to 10%;
ii) zinc oxide: 1 to 10%;
iii) butyl methoxydibenzoylmethane 0.5 to 5%
iv) benzophenones 0.5 to 10%.

According to a preferred aspect of the invention, butyl methoxydibenzoylmethane will be used in combination with octocrylene at the ratio of 1:1 or 1:1.5, the sum of the concentrations will be in the range from 1 to 10% (of the total components of the composition).

Alternatively to Octocrylene, compounds characterised by two prominent mechanisms: triplet and singlet quenching^{33,34}, may be used. Examples of said compounds include 4-Methylbezylidene Camphor, Benzophenone-3, Polysylicone 15, Polyester-8, Butyl Octyl Salicylate, Diethylhexyl-2,6-naphthalate, bis-Ethylhexyloxyphenyl Methoxyphenol triazine, Tris Tetramethyl Hydroxypiperidino citrate
1. According to a preferred aspect of the invention, zinc oxide will be used in both free and micronised form (between 20 and 200 microns). When used in micronised form, the zinc oxide will preferably be in the powder form or predispersed in solvents, preferably chosen from water, C12-C15 alkyl benzoates, mineral oil, dimethicone or triglycerides C8-C12.

According to a preferred aspect of the invention, the sum of the concentrations of diethylamine hydroxybenzoyl hexyl benzoate and zinc oxide will fall within the interval of 1 to 10% (of the total components of the composition).

In order to guarantee the maximum efficacy of the preventive/protective action of the compositions according to the invention, components with an adequate cytoprotective effect against oxidative stress, immunosuppression and DNA damage are combined with said UV-A filter systems.

According to the invention, the cytoprotective components will be chosen from the group of carboxymethyl beta-glucan and ectoine.

According to a preferred aspect of the invention, the cytoprotective components will be present in the following concentration intervals (of the total components of the composition):
i) carboxymethyl beta-glucan 0.001 to 2%;
ii) ectoine: 0.001 to 2%.

Preferably, the sum of the concentrations of beta-glucan or derivatives thereof and ectoine will preferably fall within the interval of 0.002 to 2% (of the total components of the composition).

According to the invention, the vitamin components will be chosen from the group of vitamin E, vitamin C and derivatives thereof.

Vitamin E will preferably be used in the form of natural tocopherol, synthetic tocopherol or derivatives such as tocopheryl acetate, and vitamin C will be used in the form of ascorbic acid or as ascorbic acid derivatives such as ascorbyl magnesium phosphate, ascorbyl tetraisopalmitate and sodium ascorbyl phosphate.

According to a preferred aspect of the invention, the vitamin components or derivatives thereof will be present in the following concentration intervals (of the total components of the composition):
i) Vitamin E: in concentrations at least equal to or greater than 0.1%;
ii) Vitamin C: in concentrations at least equal to or greater than 0.1%;

The sum of the concentrations of the vitamin components or derivatives thereof will preferably be at least 1% (of the total components of the composition) to ensure adequate antioxidant coverage.

According to a preferred aspect, the compositions according to the invention may also contain plant extracts such as extracts of *Vitis vinifera, Prunella vulgaris, Uncaria tormentosa, Paeonia* and *Camellia sinensis,* and substances with complementary or otherwise useful activity such as bioflavonoids, proanthocyanidins, polyphenols, verbascosides, xyloglucans or plant extracts containing them.

The compositions according to the invention will be used in medicinal products and aids, skin cosmetics and medical devices which promote a protective and preventive action against photoallergic and phototoxic skin reactions mediated by drugs, substances applied for topical or systemic use, subjective intolerance in predisposed individuals, and individuals suffering from photoinduced dermatitis.

The compositions according to the invention can be formulated according to conventional methods well known in pharmaceutical technology, such as those described in "Remington's Pharmaceutical Handbook", Mack Publishing Co., N.Y., USA, in any form designed for topical use, preferably as an emulsion with an oily or aqueous ester phase containing acrylic polymers together with other excipients in conventional use, such as preservatives, wetting agents and silicones, or in the form of a gel or emulgel, using suitable formulation agents such as carboxyvinyl and acrylic polymers, cellulose, and combinations of emulsifiers and polymers.

The following example 8E further illustrates the invention.

The terms used follow the INCI rules.

**Example 1 - Preservative Free O/W Fluid Emulsion**

| | BIOFTA001A | BIOFTA001C | | | | |
|---|---|---|---|---|---|---|
| | A | B | C | D | E | F |
| AQUA | 71.496 | 69.996 | 71.496 | 71.496 | 69.796 | 69.996 |
| PENTYLENE GLYCOL | 4 | 4 | 4 | 4 | 4 | 4 |
| SORBITOL. AQUA | 3.5 | 3.5 | 3.5 | 3.5 | 3.5 | 3.5 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| Polyglyceryl-3 Methylglucose Distearate | 4 | 4 | 4 | 4 | 4 | 4 |
| CETEARYL ALCOHOL | 1 | 1 | 1 | 1 | 1 | 1 |
| ETHYLHEXYL PALMITATE | 5 | 5 | 5 | 5 | 5 | 5 |
| PERSEA GRATISSIMA OIL | 3 | 3 | 3 | 3 | 3 | 3 |
| DIMETHICONE | 1 | 1 | 1 | 1 | 1 | 1 |
| Dipthylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELLIA SINENSIS | | | 0.2 | | 0.2 | |
| ECTOINA | | | | 0.004 | | 0.004 |
| **TOTALE** | **100** | **100** | **100** | **100** | 100 | **100** |

**Example 2 - O/W Fluid Emulsion**

| | BIOFTA001B | BIOFTA001D | | | | |
|---|---|---|---|---|---|---|
| | A | B | C | D | E | F |
| AQUA | 78.146 | 76.646 | 77.946 | 78.146 | 76.446 | 76.446 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| METHYLPARABEN | 0.2 | 0.2 | 0.2 | 0.2 | 0.2 | 0.2 |
| PROPYLPARABEN | 0.15 | 0.15 | 0.15 | 0.15 | 0.15 | 0.15 |
| PHENOSSIETHANOL | 0.5 | 0.5 | 0.5 | 0.5 | 0.5 | 0.5 |
| Polyglyceryl-3 Methylglucose Distearate | 4 | 4 | 4 | 4 | 4 | 4 |
| CETEARYL ALCOHOL | 1 | 1 | 1 | 1 | 1 | 1 |
| ETHYLHEXYL PALMITATE | 5 | 5 | 5 | 5 | 5 | 5 |
| PERSEA GRATISSIMA OIL | 3 | 3 | 3 | 3 | 3 | 3 |
| DIMETHICONE | 1 | 1 | 1 | 1 | 1 | 1 |
| Diethylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | 0.2 | 0.2 |
| ECTOINA | | | | 0.004 | | 0.004 |
| **TOTALE** | **100** | **100** | **100** | **100** | **100** | **100** |

**Example 3 - Preservative Free O/W Emulsion**

| | BIOFTA002A | BIOFTA002C | | | | |
|---|---|---|---|---|---|---|
| | A | B | C | D | E | F |
| AQUA | 72.496 | 70.996 | 72.296 | 72.496 | 70.796 | 70.996 |
| PENTYLENE GLYCOL | 4 | 4 | 4 | 4 | 4 | 4 |
| SORBITOL. AQUA | 3.5 | 3.5 | 3.5 | 3.5 | 3.5 | 3.5 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| CETEARYL OLIVATE. SORBITAN OLIVATE | 4 | 4 | 4 | 4 | 4 | 4 |
| CETEARYL ALCOHOL | 1 | 1 | 1 | 1 | 1 | 1 |
| TRITICUM VULGARE GERM OIL | 3 | 3 | 3 | 3 | 3 | 3 |
| BUTYROSPERMUM PARKII | 1 | 1 | 1 | 1 | 1 | 1 |
| COCO-CAPRYLATE CAPRATE | 3 | 3 | 3 | 3 | 3 | 3 |
| DIMETHICONE | 1 | 1 | 1 | 1 | 1 | 1 |
| Diethylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | 0.2 | |
| ECTOINA | | | | 0.004 | | 0.004 |
| **TOTALE** | **100** | **100** | **100** | **100** | **100** | **100** |

**Example 4 - O/W Emulsion**

| | BIOFTA002B | BIOFTA002D | | | | |
|---|---|---|---|---|---|---|
| | A | B | C | D | E | F |
| AQUA | 79.146 | 77.646 | 78.946 | 79.146 | 77.646 | 77.646 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| METHYLPARABEN | 0.2 | 0.2 | 0.2 | 0.2 | 0.2 | 0.2 |
| PROPYLPARABEN | 0.15 | 0.15 | 0.15 | 0.15 | 0.15 | 0.15 |
| PHENOSSIETHANOL | 0.5 | 0.5 | 0.5 | 0.5 | 0.5 | 0.5 |
| CETEARYL OLIVATE. SORBITAN OLIVATE | 4 | 4 | 4 | 4 | 4 | 4 |
| CETEARYL ALCOHOL | 1 | 1 | 1 | 1 | 1 | 1 |
| TRITICUM VULGARE GERM OIL | 3 | 3 | 3 | 3 | 3 | 3 |
| BUTYROSPERMUM PARKII | 1 | 1 | 1 | 1 | 1 | 1 |
| COCO-CAPRYLATE CAPRATE | 3 | 3 | 3 | 3 | 3 | 3 |
| DIMETHICONE | 1 | 1 | 1 | 1 | 1 | 1 |
| Diethylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | | |
| ECTOINA | | | | 0.004 | | 0.004 |
| **TOTALE** | **100** | **100** | **100** | **100** | **100** | **100** |

**Example 5 - Preservative Free ZnO O/W Emulsion**

| | BIOFTA003A | BIOFTA003C | | | | |
|---|---|---|---|---|---|---|
| | A | B | C | D | E | F |
| AQUA | 67.496 | 65.996 | 67.296 | 67.496 | 65.796 | 65.996 |
| PENTYLENE GLYCOL | 4 | 4 | 4 | 4 | 4 | 4 |
| SORBITOL. AQUA | 3.5 | 3.5 | 3.5 | 3.5 | 3.5 | 3.5 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| CETEARYL ALCOHOL. CETEARYL GLUCOSIDE | 4 | 4 | 4 | 4 | 4 | 4 |
| CERA ALBA | 1 | 1 | 1 | 1 | 1 | 1 |
| ETHYLHEXYL PALMITATE | 3 | 3 | 3 | 3 | 3 | 3 |
| COCO-CAPRYLATE CAPRATE | 3 | 3 | 3 | 3 | 3 | 3 |
| SQUALANE | 3 | 3 | 3 | 3 | 3 | 3 |
| ZINC OXIDE | 3 | 3 | 3 | 3 | 3 | 3 |
| DIMETHICONE | 1 | 1 | 1 | 1 | 1 | 1 |
| Diethylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | 0.2 | |
| ECTOINA | | | | 0.004 | | 0.004 |
| **TOTALE** | **100** | **100** | **100** | **100** | **100** | **100** |

**Example 6 - ZnO O/W Emulsion**

| | BIOFTA003B | BIOFTA003D | | | | |
|---|---|---|---|---|---|---|
| | A | B | C | D | E | F |
| AQUA | 74.146 | 72.646 | 73.946 | 74.146 | 72.446 | 72.646 |
| DISODIUM EDTA | 0.1 | 0.1 | 0.1 | 0.1 | 0.1 | 0.1 |
| GLYCERIN | 1 | 1 | 1 | 1 | 1 | 1 |
| XANTHAN GUM | 0.2 | 0.2 | 0.2 | 0.2 | 0.2 | 0.2 |
| METHYLPARABEN | 0.2 | 0.2 | 0.2 | 0.2 | 0.2 | 0.2 |
| PROPYLPARABEN | 0.15 | 0.15 | 0.15 | 0.15 | 0.15 | 0.15 |
| PHENOSSIETHANOL | 0.5 | 0.5 | 0.5 | 0.5 | 0.5 | 0.5 |
| CETEARYL ALCOHOL. CETEARYL GLUCOSIDE | 4 | 4 | 4 | 4 | 4 | 4 |
| CERA ALBA | 1 | 1 | 1 | 1 | 1 | 1 |
| ETHYLHEXYL PALMITATE | 3 | 3 | 3 | 3 | 3 | 3 |
| COCO-CAPRYLATE CAPRATE | 3 | 3 | 3 | 3 | 3 | 3 |
| SQUALANE | 3 | 3 | 3 | 3 | 3 | 3 |
| ZINC OXIDE | 3 | 3 | 3 | 3 | 3 | 3 |
| DIMETHICONE | 1 | 1 | 1 | 1 | 1 | 1 |
| Diethylamino hydroxybenzoyl hexyl benzoate | 2.5 | | 2.5 | 2.5 | | |
| OCTOCRYLENE | | 2 | | | 2 | 2 |
| BUTYL METHOXYDIBENZOYLMETHANE | | 2 | | | 2 | 2 |
| SODIUM CARBOXYMETHYL BETAGLUCAN | 0.004 | 0.004 | 0.004 | | 0.004 | |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | 0.2 | |
| ECTOINA | | | | 0.004 | | 0.004 |
| **TOTALE** | **100** | **100** | **100** | **100** | **100** | **100** |

**Example 7 - Emulgel**

| | A | B | C | D | E | F |
|---|---|---|---|---|---|---|
| AQUA | 80.976 | 79.476 | 80.776 | 80.976 | 79.476 | 79.476 |
| DIMETHICONE | 1 | 1 | 1 | 1 | 1 | 1 |
| STEARETH-10 | 0.6 | 0.6 | 0.6 | 0.6 | 0.6 | 0.6 |
| STEARETH-21 | 0.6 | 0.6 | 0.6 | 0.6 | 0.6 | 0.6 |
| TETRASODIUM EDTA | 0.1 | 0.1 | 0.1 | 0.1 | 0.1 | 0.1 |
| DICAPRYLYL CARBONATE | 3 | 3 | 3 | 3 | 3 | 3 |
| GLYCERIN | 1 | 1 | 1 | 1 | 1 | 1 |
| HELIANTHUS ANNUS (SUNFLOWER SEED) OIL | 3 | 3 | 3 | 3 | 3 | 3 |
| METHYLPARABEN | 0.2 | 0.2 | 0.2 | 0.2 | 0.2 | 0.2 |
| PROPYLPARABEN | 0.2 | 0.2 | 0.2 | 0.2 | 0.2 | 0.2 |
| PHENOXYETHANOL | 0.5 | 0.5 | 0.5 | 0.5 | 0.5 | 0.5 |
| POLYACRILAMIDE | 1.8 | 1.8 | 1.8 | 1.8 | 1.8 | 1.8 |
| C13-14 ISOPARAFFIN | 1 | 1 | 1 | 1 | 1 | 1 |
| LAURETH-7 | 0.32 | 0.32 | 0.32 | 0.32 | 0.32 | 0.32 |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | | 0.2 | | | |
| ECTOINA | | | | 0.004 | | 0.004 |
| SODIUM CARBOXYMETHYL BETAGLUCAN | 0.004 | 0.004 | 0.004 | | 0.004 | |
| OCTOCRYLENE | | 2 | | | 2 | 2 |
| BUTYL METHOXYDIBENZOYLMETHANE | | 2 | | | 2 | 2 |
| DIETHYLAMINO HYDROXYBENZOYL HEXYL BENZOATE | 2.5 | | 2.5 | 2.5 | | |
| **TOTALE** | **100.00** | **100.00** | **100.00** | **100.00** | **100.00** | **100.00** |

**Example 8 - W/O Polymeric Emulsion**

| | **A** | **B** | **C** | **D** | **E** | **F** |
|---|---|---|---|---|---|---|
| PEG-30 DIPOLYHYDROXYSTEARATE | 1.5 | 1.5 | 1.5 | 1.5 | 1.5 | 1.5 |
| CYCLOPENTASILOXANE | 4.275 | 4.275 | 4.275 | 4.275 | 4.275 | 4.275 |
| CYCLOEXASILOXANE | 0.225 | 0.225 | 0.225 | 0.225 | 0.225 | 0.225 |
| CAPRYLIC/CAPRIC TRIGLYCERIDE | 5 | 5 | 5 | | | |
| DICAPRYLYL CARBONATE | | | | 5 | 5 | 5 |
| ISOHEXADECANE | 5.91 | 5.91 | 5.91 | 2.955 | 2.955 | 2.955 |
| PPG-15 STEARYL ETHER | 0.09 | 0.09 | 0.09 | 0.045 | 0.045 | 0.045 |
| CHOLESTEROL | 0.5 | 0.5 | 0.5 | 0.5 | 0.5 | 0.5 |
| TRICONTANYL PVP | 1 | 1 | 1 | 1 | 1 | 1 |
| SODIUM CARBOXYMETHYL BETAGLUCAN | 0.004 | 0.004 | | 0.004 | 0.004 | 0.004 |
| TOCOPHERYL ACETATE | 3 | 3 | 3 | 3 | 3 | 3 |
| ASCORBYL TETRAISOPALMITATE | 0.2 | 0.2 | 0.2 | 0.2 | 0.2 | 0.2 |
| CAMELIA SINENSIS | | 0.2 | | | 0.2 | |
| ECTOINA | | | 0.004 | | 0.004 | |
| TRITICUM VULGARE | 1.5 | 1.5 | 1.5 | 1.5 | 1.5 | 1.5 |
| HYDROGENATED CASTOR OIL | 1 | 1 | 1 | 1 | 1 | 1 |
| OCTOCRYLENE | | | | | | 2 |
| BUTYL METHOXYDIBENZOYLMETHANE | | | | | | 2 |
| DIETHYLAMINO HYDROXYBENZOYL HEXYL BENZOATE | | | | 2.5 | 2.5 | |
| ZINC OXIDE | 5 | 5 | 5 | 5 | 5 | 5 |
| METHYLPARABEN | 0.15 | 0.15 | 0.15 | 0.15 | 0.15 | 0.15 |
| PROPYLPARABEN | 0.1 | 0.1 | 0.1 | 0.1 | 0.1 | 0.1 |
| GLYCERIN | 4 | 4 | 4 | 4 | 4 | 4 |
| MAGNESIUM SULPHATE | 0.7 | 0.7 | 0.7 | 0.7 | 0.7 | 0.7 |
| IMIDAZOLIDINYL UREA | 0.3 | 0.3 | 0.3 | 0.3 | 0.3 | 0.3 |
| DISODIUM EDTA | 0.1 | 0.1 | 0.1 | 0.1 | 0.1 | 0.1 |
| PANTHENOL | 0.5 | 0.5 | 0.5 | 0.5 | 0.5 | 0.5 |
| AQUA | 64.946 | 64.746 | 64.946 | 65.446 | 65.242 | 63.946 |
| **TOTALE** | **100.00** | **100.00** | **100.00** | **100.00** | **100.00** | **100.00** |

### BIBLIOGRAPHY

1. J.H. Saurat - E. Grosshans - P. Laugier - J.M. Lachapelle. Dermatologia e malattie sessualmente trasmesse - Edizioni Masson.
2. Gasparro FP. Environ Health Perspect. 2000;108(suppl 1):71-78.
3. DeBuys HV, Levy SB, Murray JC, et al. Derm Clin. 2000;18:577-590.
4. Lim HW: J Am Acad Dermatol. 2001;44:505-508.
5. Berneburg M, et al. J Biol Chem 1999; 274: 15345.
6. Pillemer L. et al. J. Biol. Chem. 137, 139-142. 1941.
7. Sherwood E. et al. J. Biol. Res. Mod. 6, 358381, 1986.
8. Zulli F. et al., Eurocosmetics, 1.1, 46-50, 1195.
9. Applegate L.A.,et al., Photochemistry and photobiology. 61, 285-291, 3, 995.
10. Zulli F.et al. International Journal of Cosmetic Science, 20, 79-86, 998.
11. Elmets C.A., et all Photoimmunol Photomed 1992; 9; 113-120, 1992.
12. Galinski, E.A.,et al., Comp. Biochem. Physiol., Vol. 117A, No. 3, 357-365, 1997.
13. Galinski, E.A., Compatible solutes of halophilic eubacteria: molecular principles, water-solute interaction, stress protection, Birkhäuser Verlag, 1993.
14. Galinski, E.A. et al., General and Applied Aspects of Halophilic Microorganisms, 1991.
15. Bunger J. et al., IFSCC Magazine vol.4, n° 2, 127-131, 2001.
16. Bunger J. et al., SPFW Journal.
17. Burton G.W. et al., Acc. Chem. Res. 19, 194202, 1986.
18. Ursini F. La Vitamina E in Dermatologia. Cleup, University Publisher, Padova, 15-34. 2001.
19. Mc Vean et al., Carcinogenesis, 18, 1617-1622, 1997.
20. Gensler L. et al. Nutr. Cancer, 15, 97107, 1991.
21. Berton T.R. et al., Molec. Carcinogen., 23, 175-184, 1998.
22. Halliday GM et al., Australas J Dermatol 1998 May;39(2):71-5.
23. Padh H Nutr. Rev. 49, 65-70, 1991.
24. Niki E. et al.: J. Biol. Chem. 259, 4177-4182, 1984.
25. Leung H. W. et al., Biochim. Biophys. Acta 664, 266-272, 1981.
26. Harman D.: The aging process. Proc. Natl. Acad. Sci. USA 78, 7124-7128, 1981.
27. Ames B.N. Free Rad. Res. Commun. 7, 121128, 1989.
28. Halliwell B. and Gutteridge J.M.C.: Free Radicals in Biology and Medicine (Clarendon, Oxford) 2nd Ed., 1990.
29. Fraga C.G.,et al.: Ascorbic acid protects against endogenous oxidative.
30. Leung A., Foster S. Encyclopedia of common natural ingredients, 2° edition. A. Wiley Interscience Pubblication 1996.
31. Firenzuoli F. Fitoperapia 3° edition. Edizioni Masson, 2002.
32. Psotova J, et al., J. Photochem Photobiol B. 2006 Sep 1;84(3):167-74. Epub 2006 Apr 21.
33. Christine Mendrock-Edinger et al. The quest for Avobenzone Stabilizers and sunscreen photostability. C&T, 124/n°2. 2009.
34. Bonda C. Quenching excited state. C&T, 123/n°2. 2008
35. US 7 008 627 B1
36. FR 2 865 398
37. DE 199 33 466

## Claims

1. Use of a combination of:
a) UV-A photoabsorbent systems;
b) carboxymethyl beta-glucan and ectoine as cytoprotective agents;
c) antioxidants selected from vitamins C and/or E and/or derivatives thereof;
d) and possibly bioflavonoids, proanthocyanidins, polyphenols, verbascosides, xyloglucans or plant extracts containing them;
to prepare topical compositions for the prevention of and protection against photoallergic and phototoxic skin reactions mediated by pharmaceuticals, topical products and/or foods, or caused by subjective predispositions.

2. Use as claimed in claim 1, wherein the UV-A photoabsorbent components are selected from diethylamine hydroxybenzoyl hexyl benzoate, zinc oxide, butyl methoxydibenzoylmethane, benzophenones.

3. Use as claimed in claim 2, wherein the photoabsorbent components are present in the following concentration intervals:
i) diethylamine hydroxybenzoyl hexyl benzoate: 0.5 to 10%;
ii) zinc oxide 1 to 10%;
iii) butyl methoxydibenzoylmethane: 0.5 to 5%;
iv) benzophenones: 0.5 to 10%;
and the sum of the concentrations of diethylamine hydroxybenzoyl hexyl benzoate and zinc oxide falls into an interval between 1 and 10%, the concentrations being expressed as a proportion of the total components of the composition.

4. Use as claimed in claim 3, wherein butyl methoxydibenzoyl-methane is used in combination with Octocrylene or another stabilizing agent **characterised by** triplet and singlet quenching in the ratio of 1:1 or 1:1.5, the sum of the concentrations falling into the interval between 1 and 10% (of the total components of the composition).

5. Use as claimed in claim 1, wherein zinc oxide is used in free form or micronised between 20 and 200 microns, as a powder or predispersed in solvents chosen from water, C12-C15 alkyl benzoates, mineral oil, dimethicone or triglycerides C8-C12.

6. Use as claimed in claim 1, wherein the concentration of carboxymethyl beta-glucan and ectoine is 0.001 to 2% and 0.001 to 2% respectively, and their sum falls into the interval between 0.002 and 2%, the concentrations being expressed as a proportion of the total components of the composition.

7. Use as claimed in claim 1, wherein vitamin E is present in the form of natural or synthetic tocopherol or derivatives thereof, in concentrations at least equal to or greater than 2%, and vitamin C is present in the form of ascorbic acid or derivatives thereof chosen from ascorbyl magnesium phosphate, ascorbyl tetraisopalmitate and sodium ascorbyl phosphate, in concentrations at least equal to or greater than 0.1%, and the sum of the concentrations of vitamin E and vitamin C is at least greater than 1%, the concentrations being expressed as a proportion of the total components of the composition.

8. Use as claimed in any of claims 1 to 7, also containing plant extracts such as extracts of *Vitis vinifera*, *Prunella vulgaris*, *Uncaria tormentosa*, *Paeonia* and *Camellia Sinensis.*

9. Use as claimed in any of claims 1 to 8, also containing substances such as bioflavonoids, proanthocyanidins, polyphenols, verbascosides, xyloglucans or plant extracts containing them.

10. Use as claimed in any of claims 1 to 9, in the form of an emulsion with an oily or aqueous ester phase, or in the form of a gel or emulgel.

## Patentansprüche

1. Verwendung einer Kombination von:
a) UV-A-photoabsorbierenden Systemen;
b) Carboxymethyl-beta-glucan und Ectoin als zytoprotektive Agentien;
c) Antioxidantien, ausgewählt aus Vitaminen C und/oder E und/oder Derivaten davon;
d) und gegebenenfalls Bioflavonoiden, Proanthocyanidinen, Polyphenolen, Verbascosiden, Xyloglucanen oder Pflanzenextrakten, die diese enthalten;
zur Herstellung topischer Zusammensetzungen für die Prävention von und den Schutz gegen photoallergische und phototoxische Hautreaktionen, die von Pharmazeutika, topischen Produkten und/oder Lebensmitteln vermittelt werden, oder durch persönliche Prädispositionen verursacht werden.

2. Verwendung nach Anspruch 1, wobei die UV-A-photoabsorbierenden Komponenten ausgewählt sind aus Diethylaminhydroxybenzoylhexylbenzoat, Zinkoxid, Butylmethoxydibenzoylmethan, Benzophenonen.

3. Verwendung nach Anspruch 2, wobei die photoabsorbierenden Komponenten in den folgenden Konzentrationsintervallen vorliegen:
i) Diethylaminhydroxybenzoylhexylbenzoat: 0,5 bis 10%;
ii) Zinkoxid 1 bis 10%;
iii) Butylmethoxydibenzoylmethan: 0,5 bis 5%;
iv) Benzophenone: 0,5 bis 10%;
und die Summe der Konzentrationen von Diethylaminhydroxybenzoylhexylbenzoat und Zinkoxid in einem Intervall zwischen 1 und 10% liegt, wobei die Konzentrationen als Proportion der gesamten Komponenten der Zusammensetzung angegeben sind.

4. Verwendung nach Anspruch 3, wobei Butylmethoxydibenzoylmethan in Kombination mit Octocrylen oder einem anderen stabilisierenden Agens verwendet wird, das **gekennzeichnet ist durch** Triplett- und Singulett-Quenchen im Verhältnis 1:1 oder 1:1,5, wobei die Summe der Konzentrationen in das Intervall zwischen 1 und 10% (der gesamten Komponenten der Zusammensetzung) fällt.

5. Verwendung nach Anspruch 1, wobei Zinkoxid in freier Form verwendet wird oder mikronisiert zwischen 20 und 200 Micron, als ein Pulver oder prädispergiert in Solventien ausgewählt aus Wasser, C12 - C15-Alkylbenzoaten, Mineralöl, Dimethicon oder Triglyceriden C8-C12.

6. Verwendung nach Anspruch 1, wobei die Konzentration von Carboxymethyl-betaglucan und Ectoin zwischen 0,001 bis 2% bzw. 0,001 bis 2% beträgt, und ihre Summe in das Intervall zwischen 0,002 und 2% fällt, wobei die Konzentrationen als eine Proportion der gesamten Komponenten der Zusammensetzung angegeben sind.

7. Verwendung nach Anspruch 1, wobei Vitamin E in der Form natürlichen oder synthetischen Tocopherols oder Derivaten davon, in Konzentrationen von mindestens gleich oder mehr als 2% vorliegt, und Vitamin C in Form von Ascorbinsäure oder Derivaten davon, die ausgewählt sind aus Ascorbylmagnesiumphosphat, Ascorbyltetraisopalmitat und Natriumascorbylphosphat, in Konzentrationen von mindestens gleich oder mehr als 0,1% vorliegt, und die Summe der Konzentrationen von Vitamin E und Vitamin C mindestens größer als 1% ist, wobei die Konzentrationen als Proportion der gesamten Komponenten der Zusammensetzung angegeben sind.

8. Verwendung nach einem der Ansprüche 1 bis 7, ebenfalls enthaltend Pflanzenextrakte wie Extrakte von *Vitis vinifera, Prunella vulgaris, Uncaria tormentosa, Paeonia* und *Camellia Sinensis.*

9. Verwendung nach einem der Ansprüche 1 bis 8, ebenfalls enthaltend Substanzen wie Bioflavonoide, Proanthocyanidine, Polyphenole, Verbascoside, Xyloglucane oder Pflanzenextrakte, die diese enthalten.

10. Verwendung nach einem der Ansprüche 1 bis 9, in Form einer Emulsion mit einer öligen oder wässrigen Esterphase, oder in Form eines Gels oder Emulgels.

## Revendications

1. Utilisation d'une combinaison :
a) de systèmes photoabsorbants du rayonnement UV-A ;
b) d'un carboxyméthyl bêta-glucane et d'ectoïne comme agents cytoprotecteurs ;
c) d'antioxydants sélectionnés parmi les vitamines C et/ou E et/ou leurs dérivés ;
d) et de manière possible de bioflavonoïdes, de proanthocyanidines, de polyphénols, de verbascosides, de xyloglucanes ou d'extraits végétaux les contenant ;
pour préparer des compositions topiques destinées à la prévention et à la protection vis-à-vis des réactions cutanées photoallergiques et phototoxiques à médiation par des substances pharmaceutiques, des produits topiques et/ou des aliments, ou provoquées par des prédispositions subjectives.

2. Utilisation telle que revendiquée selon la revendication 1, où les composants photoabsorbants du rayonnement UV-A sont sélectionnés parmi le benzoate de diéthylamine hydroxybenzoyl hexyle, l'oxyde de zinc, le butyl méthoxydibenzoylméthane, les benzophénones.

3. Utilisation telle que revendiquée selon la revendication 2, où les composants photoabsorbants sont présents selon les intervalles de concentration suivants :
i) benzoate de diéthylamine hydroxybenzoyl hexyle : 0,5 à 10 % ;
ii) oxyde de zinc 1 à 10 % ;
iii) butyl méthoxydibenzoylméthane : 0,5 à 5 % ;
iv) benzophénones : 0,5 à 10 % ;
et la somme des concentrations du benzoate de diéthylamine hydroxybenzoyl hexyle et de l'oxyde de zinc tombe dans un intervalle compris entre 1 et 10 %, les concentrations étant exprimées sous la forme d'une proportion des composants totaux de la composition.

4. Utilisation telle que revendiquée selon la revendication 3, où le butyl méthoxydibenzoyl-méthane est utilisé en combinaison avec l'octocrylène ou un autre agent stabilisant **caractérisé par** le piégeage des triplets et des singulets en le rapport de 1:1 ou 1:1,5, la somme des concentrations tombant dans l'intervalle compris entre 1 et 10 % (des composants totaux de la composition).

5. Utilisation telle que revendiquée selon la revendication 1, où l'oxyde de zinc est utilisé sous la forme libre ou micronisée entre 20 et 200 microns, sous la forme d'une poudre ou prédispersée dans les solvants choisis parmi l'eau, les benzoates d'alkyle en C₁₂ à C₁₅, l'huile minérale, la diméthicone ou les triglycérides en C₈ à C₁₂.

6. Utilisation telle que revendiquée selon la revendication 1, où la concentration en carboxyméthyl bêta-glucane et ectoïne est respectivement de 0,001 à 2 % et 0,001 à 2 %, et leur somme tombe dans l'intervalle compris entre 0,002 et 2 %, les concentrations étant exprimées sous la forme d'une proportion des composants totaux de la composition.

7. Utilisation telle que revendiquée selon la revendication 1, où la vitamine E est présente so us la forme de tocophérol d'origine naturelle ou synthétique ou ses dérivés, en des concentrations au moins égales ou supérieures à 2 %, et la vitamine C est présente sous la forme d'acide ascorbique ou de ses dérivés choisis parmi le phosphate d'ascorbyl magnésium, le tétraisopalmitate d'ascorbyle et le phosphate d'ascorbyle sodique, en des concentrations au moins égales ou supérieures à 0,1 %, et la somme des concentrations de la vitamine E et de la vitamine C est au moins supérieure à 1 %, les concentrations étant exprimées sous la forme d' une proportion des composants totaux de la composition.

8. Utilisation telle que revendiquée selon l'une quelconque des revendications 1 à 7, contenant également des extraits végétaux tels que des extraits de *Vitis vinifera, Prunella vulgaris, Uncaria tormentosa, Paeonia* et de *Camellia Sinensis.*

9. Utilisation telle que revendiquée selon l'une quelconque des revendications 1 à 8, contenant également des substances telles que des bioflavonoïdes, des proanthocyanidines, des polyphénols, des verbascosides, des xyloglucanes ou des extraits végétaux les contenant.

10. Utilisation telle que revendiquée selon l'une quelconque des revendications 1 à 9, sous la forme d'une émulsion avec une phase est er huileuse ou aqueuse, ou sous la forme d'un gel ou d'un émulgel.
